(19) Europäisches Patentamt
European Patent Office
Office européen des brevets

(11) Veröffentlichungsnummer: **0 049 416**
**B1**

(12) # EUROPÄISCHE PATENTSCHRIFT

(45) Veröffentlichungstag der Patentschrift: **11.07.84**

(51) Int. Cl.³: **C 07 D 253/06**, A 01 N 43/64

(21) Anmeldenummer: **81107511.8**

(22) Anmeldetag: **22.09.81**

(54) **Substituierte 6-Halogen-tert.-butyl-1,2,4-triazin-5-one, Verfahren zu ihrer Herstellung sowie ihre Verwendung als Herbizide.**

(30) Priorität: **02.10.80 DE 3037300**

(43) Veröffentlichungstag der Anmeldung: **14.04.82 Patentblatt 82/15**

(45) Bekanntmachung des Hinweises auf die Patenterteilung: **11.07.84 Patentblatt 84/28**

(84) Benannte Vertragsstaaten: **AT BE CH DE FR GB IT LI NL SE**

(56) Entgegenhaltungen:
**DE - A - 2 165 554**
**DE - A - 2 726 016**
**FR - A - 1 577 658**
**GB - A - 1 182 801**
**GB - A - 1 182 802**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Kranz, Eckart, Dr., Am Acker 9, D-5600 Wuppertal 1 (DE)**
Erfinder: **Findeisen, Kurt, Dr., In der Follmühle 10, D-5068 Odenthal 2 (DE)**
Erfinder: **Schmidt, Robert, Dr., Im Waldwinkel 110, D-5060 Bergisch-Gladbach 2 (DE)**
Erfinder: **Eue, Ludwig, Dr., Paul-Klee-Strasse 36, D-5090 Leverkusen 1 (DE)**

EP 0 049 416 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue 6-Halogen-tert.-butyl-1,2,4-triazin-5-one, ein Verfahren zu ihrer Herstellung sowie ihre Verwendung als Herbizide, insbesondere als selektive Herbizide.

Es ist bereits bekannt geworden, dass substituierte 1,2,4-Triazin-5-one, wie insbesondere das 4-Amino-3-methylthio-6-tert.-butyl-1,2,4-triazin-5-on, als Herbizide verwendet werden können (vgl. z.B. DE-C 1795784, GB-A 1182801, GB-A 1182802 und US-A 3671523). Aus der gleichen Stoffgruppe ist weiterhin das 4-Amino-3-methyl- thio-6-(2,2-dichlor-1-methyl-cyclopropyl)-1,2,4-triazin-5-on als herbizid wirksame Verbindung bekannt geworden (vgl. DE-A 2726016). Ferner ist es bekannt, dass auch bestimmte 4-alkylsubstituierte 1,2,4-Triazin-5-one, wie z.B. das 4-Methyl-3-methylthio-6-isopropyl-1,2,4-triazin-5-on, herbizide Eigenschaften aufweisen (vgl. FR-A 1577658). In bestimmten Kulturen ist jedoch ein selektiver Einsatz der vorbekannten Triazinone nicht möglich, da es infolge der durchweg hohen herbiziden Potenz dieser Stoffgruppe auch bei bestimmten Nutzpflanzen zu Schäden kommen kann; die Verträglichkeit gegenüber den vorbekannten Triazinonen ist demnach bei verschiedenen Nutzpflanzen nicht ausreichend.

Es wurden neue substituierte 6-Halogen-tert.-butyl-1,2,4-triazin-5-one der allgemeinen Formel

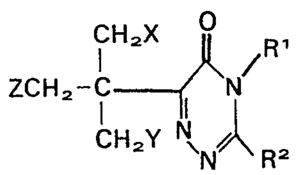

(I)

in welcher

$R^1$ für Amino oder Methyl steht,

$R^2$ für $C_{1-4}$-Alkylmercapto, $C_{1-4}$-Alkylamino oder Di-$(C_{1-4})$-alkylamino steht,

X für Halogen steht, und

Y und Z für Wasserstoff oder Halogen stehen,

gefunden.

Weiterhin wurde gefunden, dass man die substituierten 6-Halogen-tert.-butyl-1,2,4-triazon-5-one der Formel (I) erhält, wenn man in einer ersten Stufe Halogenpivaloylcyanide der Formel

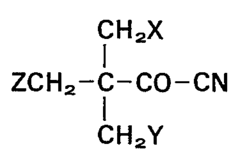

(II)

in welcher

X, Y und Z die oben angegebene Bedeutung haben,

gegebenenfalls in Gegenwart einer flüssigen Carbonsäure als Lösungsmittel, mit einer anorganischen Säure umsetzt und die hierbei entstehenden halogenierten Trimethylbrenztraubensäureamide der Formel

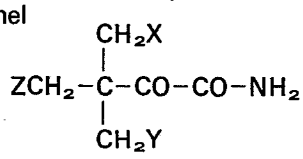

(IIIa)

in welcher

X, Y und Z die oben angegebene Bedeutung haben,

in einer zweiten Stufe in an sich bekannter Weise entweder direkt in der anfallenden Lösung oder nach Zwischenisolierung, gegebenenfalls nach vorheriger Verseifung zu den freien halogenierten Trimethylbrenztraubensäuren der Formel

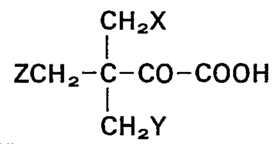

(IIIb)

in welcher

X, Y und Z die oben angegebene Bedeutung haben,

mit Verbindungen der Formel

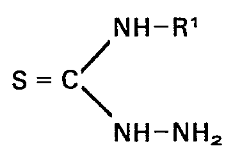

(IV)

in welcher

$R^1$ die oben angegebene Bedeutung hat,

in wässriger bzw. in wässrig-saurer Lösung, gegebenenfalls in Gegenwart eines organischen Verdünnungsmittels, zu 6-Halogen-tert.-butyl-3-mercapto-1,2,4-triazin-5-onen der Formel

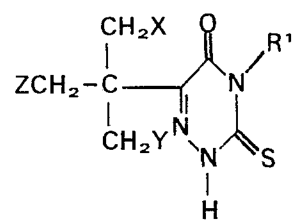

(V)

in welcher

$R^1$, X, Y und Z die oben angegebene Bedeutung haben,

umsetzt, diese in einer *dritten Stufe* in bekannter Weise in alkalischer Lösung mittels Alkylhalogenid, vorzugsweise Alkyljodid oder -bromid, zu 6-Halogen-tert.-butyl-1,2,4-triazin-5-onen der Formel

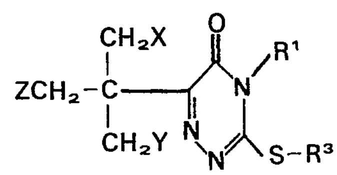

(Ia)

in welcher

$R^1$, X, Y und Z die oben angegebene Bedeutung haben und

$R^3$ für Alkyl, vorzugsweise mit 1 bis 4 Kohlenstoffatomen, steht,

alkyliert und diese gegebenenfalls in einer vierten Stufe mit Aminen der Formel

$$HNR^4R^5 \qquad (VI)$$

in welcher

$R^4$ für Wasserstoff oder Alkyl, vorzugsweise mit 1 bis 4 Kohlenstoffatomen, steht, und

$R^5$ für Alkyl, vorzugsweise mit 1 bis 4 Kohlenstoffatomen, steht,

in Gegenwart eines Verdünnungsmittels umsetzt.

Ausserdem wurde gefunden, dass die substituierten 6-Halogentert.-butyl-1,2,4-triazin-5-one der Formel (I) gute herbizide, insbesondere selektiv-herbizide Eingenschaften aufweisen.

Überraschenderweise zeigen die erfindungsgemässen Verbindungen gegenüber dem bekannten 4-Amino-3-methylthio-6-tert.-butyl-1,2,4-triazin-5-on bei gleich guter allgemeiner herbizider Wirkung eine deutlich bessere Verträglichkeit bei wichtigen Kulturpflanzen, wie insbesondere bei Mais, Baumwolle und Getreide. Auch im Vergleich zu den übrigen vorbekannten Triazinonen zeigen die erfindungsgemässen Verbindungen eine überlegene Wirksamkeit, insbesondere eine bessere Selektivität. Die erfindungsgemässen Wirkstoffe stellen somit eine wesentliche Bereicherung der herbiziden Mittel, insbesondere der selektiven chemischen Unkrautbekämpfung dar.

Die erfindungsgemässen substituierten 6-Halogen-tert.-butyl-1,2,4-triazin-5-one sind durch die Formel (I) allgemein definiert. In dieser Formel steht $R^2$ vorzugsweise für Methyl-, Ethyl- oder Propylthio, ferner für Methyl-, Ethyl-, oder Propylthio, ferner für Methyl-, Ethyl-, Dimethyl-, Diethyl- sowie Ethylmethylamino; X steht vorzugsweise für Fluor, Chlor oder Brom; Y und Z stehen vorzugsweise für Wasserstoff, Fluor, Chlor oder Brom.

Verwendet man beispielsweise Chlorpivaloylcyanid als Ausgangsstoff in der ersten Stufe, setzt in der zweiten Stufe die entsprechende freie Säure mit Thiocarbohydrazid um und verwendet in der dritten Stufe Methylbromid als Alkylierungsmittel, so kann der Reaktionsverlauf durch das folgende Formelschema wiedergegeben werden:

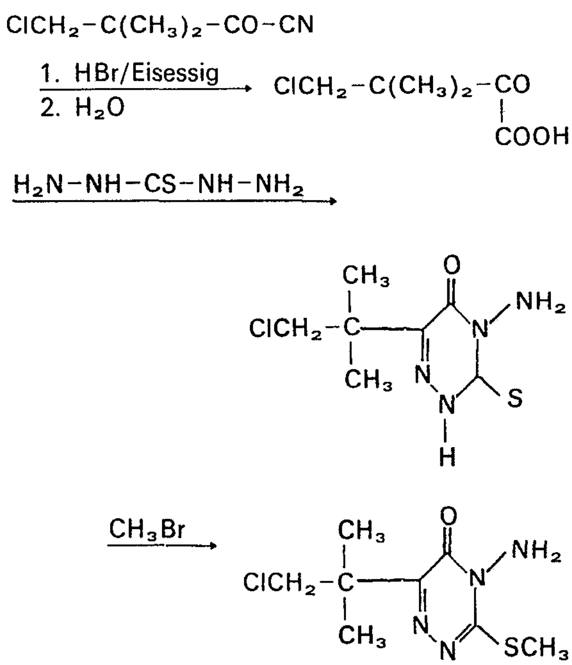


Verwendet man das so erhaltene 4-Amino-6-chlor-tert.-butyl-3-methylthio-1,2,4-triazin-5-on und Dimethylamin als Ausgangsstoffe für die vierte Stufe, so kann der Reaktionsablauf durch das folgende Formelschema wiedergegeben werden:

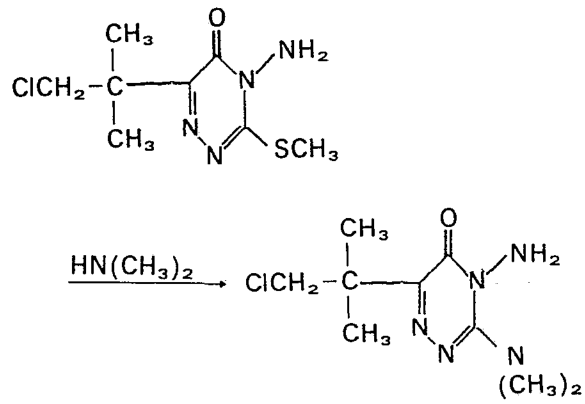


Die für das erfindungsgemässe Verfahren als Ausgangsstoffe zu verwendenden Halogenpivaloylcyanide sind durch die Formel (II) allgemein definiert. In dieser Formel stehen X, Y und Z vorzugsweise für die Reste, die bereits im Zusammenhang mit der Beschreibung der erfindungsgemässen Stoffe der Formel (I) vorzugsweise für diese Substituenten genannt wurden.

Die Halogenpivaloylcyanide der Formel (II) sind noch nicht bekannt. Sie werden erhalten, indem man die entsprechenden Halogenpivaloylhalogenide bzw. -anhydride der Formeln

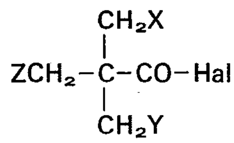 (VIIa)

bzw.

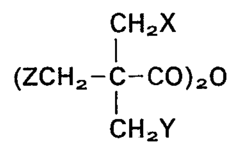 (VIIb)

in welchen

Hal für Halogen, vorzugsweise Chlor oder Brom, steht, und

X, Y und Z die oben angegebene Bedeutung haben,

mit Trimethylsilylcyanid gegebenenfalls in Gegenwart eines Verdünnungsmittels umsetzt. Trimethylsilylcyanid, $(CH_3)_3Si-CN$, ist bekannt (vgl. z.B. „Synthesis" 1979, Seiten 522 und 523).

Dieses Verfahren zur Herstellung der Halogenpivaloylcyanide der Formel (II) wird gegebenenfalls in Gegenwart eines Verdünnungsmittels durchgeführt. Hierzu gehören vorzugsweise inerte organische Lösungsmittel, wie Ketone, z.B. Aceton und Methylethylketon; Nitrile, z.B. Acetonitril; Ether, z.B. Tetrahydrofuran oder Dioxan; sowie aromatische Kohlenwasserstoffe, z.B. Benzol, Toluol oder Xylol.

Vorzugsweise wird ohne Lösungsmittel gearbeitet.

Die Reaktionstemperaturen können bei der Durchführung dieses Verfahrens in einem grösseren Bereich variiert werden. Im allgemeinen arbeitet man zwischen 50 und 250° C, vorzugsweise zwischen 80 und 180° C.

Bei der Durchführung dieses Verfahrens arbeitet

man vorzugsweise mit äquivalenten Mengen an Ausgangsstoffen. Die Isolierung der Verbindungen der Formel (II) erfolgt in üblicher Weise.

Die Halogenpivaloylhalogenide bzw. -anhydride der Formeln (VIIa) bzw. (VIIb) sind bekannt, bzw. können sie in allgemein bekannter Art und Weise erhalten werden.

Die erste Stufe des erfindungsgemässen Verfahrens kann in Abwesenheit oder in Gegenwart einer unter den Reaktionsbedingungen flüssigen, niederaliphatischen Carbonsäure als Lösungsmittel durchgeführt werden. Als solche Lösungsmittel kommen vorzugsweise Carbonsäuren mit 1 bis 6 Kohlenstoffatomen, wie Essigsäure, Propionsäure oder Ameisensäure, in Frage.

Die erste Stufe des erfindungsgemässen Verfahrens wird mit Hilfe einer starken anorganischen Säure durchgeführt. Hierzu gehören vorzugsweise die Halogenwasserstoffsäuren, wie Chlorwasserstoff und Bromwasserstoff, sowie konzentrierte Schwefelsäure.

Die Reaktionstemperaturen können bei der Durchführung dieser Verfahrensstufe in einem grösseren Bereich variiert werden. Im allgemeinen arbeitet man zwischen −20 und +50° C, vorzugsweise zwischen 0 und +40° C.

Bei der Durchführung der ersten Stufe des erfindungsgemässen Verfahrens setzt man auf 1 mol Halogenpivaloylcyanid der Formel (II) vorzugsweise 1 bis 10 mol an anorganischer Säure ein.

Die zweite Stufe des erfindungsgemässen Verfahrens erfolgt vorzugsweise ohne vorherige Zwischenisolierung der halogenierten Trimethylbrenztraubensäureamide der Formel (IIIa) und nach Verseifung derselben in üblicher Weise zu freien Säuren der Formel (IIIb).

Die zweite Stufe des erfindungsgemässen Verfahrens wird in wässeriger bzw. in Gegenwart einer wässerig-sauren Lösung, wie einer halogenwasserstoffsauren, vorzugsweise einer salzsauren, oder einer schwefelsauren Lösung durchgeführt.

Wird in Gegenwart eines organischen Lösungsmittels gearbeitet, so kommen alle üblichen organischen Lösungsmittel in frage, wie insbesondere Dimethylformamid.

Die Reaktionstemperaturen können bei der Durchführung der zweiten Stufe des erfindungsgemässen Verfahrens in einem grösseren Bereich variiert werden. Im allgemeinen arbeitet man zwischen 0 und 120° C, vorzugsweise zwischen 0 und 100° C.

Bei der Durchführung der zweiten Stufe des erfindungsgemässen Verfahrens setzt man die Ausgangsstoffe vorzugsweise in molaren Mengen bzw. mit einem Überschuss an Verbindungen der Formel (IV) ein. Die Isolierung der Zwischenprodukte der Formel (V) erfolgt in üblicher Weise.

Die dritte Stufe des erfindungsgemässen Verfahres wird in Gegenwart einer Base durchgeführt. Hierbei werden vorzugsweise Alkalihydroxide, wie Natriumhydroxid, in wässeriger Lösung oder Alkalialkoholate, wie Natriummethylat, wobei, überschüssiger Alkohol als Lösungsmittel verwendet wird, eingesetzt.

Die Reaktionstemperaturen können bei der Durchführung der dritten Stufe des erfindungsgemässen Verfahrens in einem grösseren Bereich variiert werden. Im allgemeinen arbeitet man zwischen 0 und 100° C, vorzugsweise zwischen 0 und 50° C.

Bei der Durchführung der dritten Stufe des erfindungsgemässen Verfahrens setzt man auf 1 mol Zwischenprodukt der Formel (V) vorzugsweise 1 bis 1,5 mol Alkylierungsmittel ein. Die Isolierung der Zwischenprodukte bzw. Endprodukte der Formel (Ia) erfolgt in üblicher Weise.

Als Verdünnungsmittel kommen für die vierte Stufe des erfindungsgemässen Verfahrens vorzugsweise organische Lösungsmittel in Frage, wie insbesondere Isopropanol/Eisessig.

Die Reaktionstemperaturen können bei der Durchführung der vierten Stufe des erfindungsgemässen Verfahrens in einem grösseren Bereich variiert werden. Im allgemeinen arbeitet man zwischen 20 und 180° C, vorzugsweise zwischen 40 und 150° C.

Bei der Durchführung der vierten Stufe des erfindungsgemässen Verfahrens setzt man auf 1 mol der Verbindung der Formel (Ia) vorzugsweise 1 bis 3 mol Amin der Formel (VI) ein. Die Isolierung der Endprodukte erfolgt in üblicher Weise.

Die erfindungsgemässen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemässen Stoffe als totale oder selektive Herbizide wirken, hängt im wesentlichen von der angewendeten Menge ab.

Die erfindungsgemässen Wirkstoffe können z.B. bei den folgenden Pflanzen verwendet werden:

Dikotyle Unkräuter der Gattungen: *Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.*

Dicotyle Kulturen der Gattungen: *Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cuburbita.*

Monokotyle Unkräuter der Gattungen: *Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.*

Monokotyle Kulturen der Gattungen: *Oryza, Zea, Triticum, Hordeum, Avena, Secale, Sorghum, Panicum, Saccharum, Ananas, Asparagus, Allium.*

Die Verwendung der erfindungsgemässen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemässen Wirkstoffe zeigen neben einer sehr guten allgemeinen herbiziden Wirkung eine gute Verträglichkeit gegenüber Nutzpflanzen. So ist er möglich, wichtige Schadgräser selektiv in wichtigen Kulturpflanzen, wie z.B. in Mais, Soja, Baumwolle und Getreide zu bekämpfen.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone wie Aceton, Methyläthylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Alminiumoxid und Silicate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und antiionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfrass, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Giessen, Spritzen, Sprühen, Streuen.

Die erfindungsgemässen Wirkstoffe können sowohl vor als auch nach dem Auflaufen der Pflanzen appliziert werden. Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann in grösseren Bereichen schwanken. Sie hängt im wesentlichen von der Art des gewünschten Effekts ab. Im allgemeinen liegen die Aufwandmengen zwischen 0,1 und 10 kg Wirkstoff pro ha, vorzugsweise zwischen 0,1 und 5 kg/ha.

Die nachfolgenden Beispiele dienen zur weiteren Erläuterung der Erfindung.

*Herstellungsbeispiele*

*Beispiel 1*

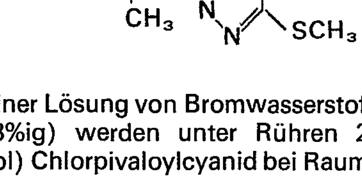

(1)

Zu 9 l einer Lösung von Bromwasserstoff in Eisessig (33%ig) werden unter Rühren 2,31 kg (15,88 mol) Chlorpivaloylcyanid bei Raumtempe-

ratur gegeben. Man lässt 4 Stunden bei Raumtemperatur nachrühren. Anschliessend werden bei 7 bis 10°C 288 ml (15,88 mol) Wasser zugegeben (exotherme Reaktion, ca. 37°C) und 3 Stunden bei Raumtemperatur nachgerührt. Die Reaktionslösung wird danach bei 7 bis 10°C in eine Mischung von 2,03 kg Thiocarbohydrazid und 15,9 l in 1N-Salzsäure eingetragen (stark exotherme Reaktion). Dieses Reaktionsgemisch wird 2 Stunden bei 7 bis 10°C und 14 Stunden bei Raumtemperatur nachgerührt. Danach wird das ausfallende Kristallisat abfiltriert, mit Wasser gewaschen und getrocknet. Man erhält 2995 g (80,4% der Theorie) rohes 4-Amino-6-chlor-tert.-butyl-3-mercapto-1,2,4-triazin-5-on vom Schmelzpunkt 202-208°C.

2813,4 g (12 mol) des so erhaltenen 4-Amino-6-chlor-tert.-butyl-3-mercapto-1,2,4-triazin-5-ons werden in 15 l 1N-Natronlauge gelöst. Nach vollständiger Lösung des Produktes werden bei 7 bis 10°C 1873,6 g Methyljodid zugetropft. Nach beendeter Zugabe lässt man 2 Stunden bei 7 bis 10°C und über Nacht bei Raumtemperatur nachrühren. Danach wird der entstandene Feststoff abgesaugt, mit Wasser gewaschen und getrocknet. Man erhält 1938 g (65% der Theorie) 4-Amino-6-chlor-tert.-butyl-3-methylthio-1,2,4-triazin-5-on vom Schmelzpunkt 98°C.

*Herstellung des Ausgangsproduktes*

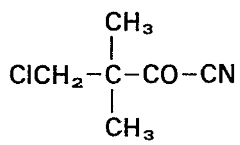

8300 g (49,5 mol) 92,5%iges β-Chlorpivaloylchlorid werden auf 100°C erwärmt und innerhalb von etwa 2 Stunden mit 4950 g (50 mol) Trimethylsilylcyanid versetzt. Das entstehende Trimethylsilylchlorid wird gleichzeitig abdestilliert. Nach beendeter Zugabe wird die Temperatur langsam auf 140°C gesteigert und etwa 1,5 Stunden bei dieser Temperatur gerührt. Anschliessend wird die Reaktionsmischung im Vakuum destilliert. Man erhält 7500 g β-Chlorpivaloylcyanid vom Siedepunkt 62-65°C/16 mbar.

*Beispiel 2*

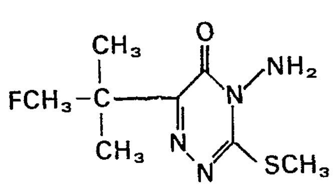

(2)

Entsprechend Beispiel 1 erhält man, ausgehend von Fluorpivaloylcyanid, in 80%iger Ausbeute zunächst rohes 4-Amino-6-fluor-tert.-butyl-3-mercapto-1,2,4-triazin-5-on und durch weitere Umsetzung mit Methyljodid ebenfalls in 80%iger Ausbeute 4-Amino-6-fluor-tert.-butyl-3-methylthio-1,2,4-triazin-5-on vom Schmelzpunkt 121-122°C.

*Beispiel 3*

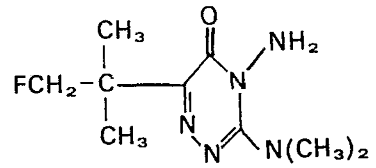

(3)

13,6 g (0,3 mol) Dimethylamin werden unter Eiskühlung in eine Mischung von 350 ml Isopropanol und 12 g Eisessig eingeleitet. Diese Reaktionslösung wird anschliessend mit 21,8 g (0,1 mol) 4-Amino-6-fluor-tert.-butyl-3-methylthio-1,2,4-triazin-5-on (Beispiel 2) versetzt. Man lässt auf Raumtemperatur erwärmen und erhitzt anschliessend 15 Stunden unter Rückfluss. Danach wird das Reaktionsgemisch eingeengt und der ölige Rückstand in Wasser eingeerührt. Man extrahiert mit Methylenchlorid, trocknet über Natriumsulfat und engt ein. Der ölige Rückstand wird in Diisopropylether aufgenommen, angeimpft und der auskristallisierende Feststoff abgesaugt und getrocknet. Man erhält 15,9 g (69% der Theorie) 4-Amino-3-dimethylamino-6-fluor-tert.-butyl-1,2,4-triazin-5-on vom Schmelzpunkt 86-87°C.

*Beispiel 4*

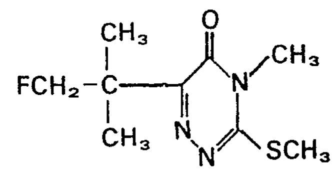

(4)

Zu 92,8 g einer Lösung von Bromwasserstoff in Eisessig (33%ig) werden unter Rühren 15 g (0,116 mol) Fluorpivaloylcyanid bei Raumtemperatur zugetropft. Nach beendeter Zugabe lässt man 2 Stunden bei Raumtemperatur nachrühren. Anschliessend tropft man 1,1 g (0,06 mol) Wasser zu (exotherme Reaktion) und rührt 3 Stunden bei Raumtemperatur nach. Danach wird bei 30°C im Vakuum eingeengt. Der Rückstand wird in 23 ml Dimethylformamid gelöst und unter Rückfluss zu 12,8 g (0,116 mol) 4-Methylthiosemicarbazid in 232 ml Wasser gegeben. Man lässt 48 Stunden unter Rückfluss rühren und saugt anschliessend das ausgefallene Kristallisat ab. Nach Verreiben mit Ligroin/Petrolether und säulenchromatographischer Reinigung erhält man 11 g (44% der Theorie) 6-Fluor-tert.-butyl-3-mercapto-4-methyl-1,2,4-triazin-5-on vom Schmelzpunkt 179-180°C.

5,4 g (0,024 mol) des so erhaltenen 6-Fluor-tert.-butyl-3-mercapto-4-methyl-1,2,4-triazin-5-ons werden in 0,96 g (0,024 mol) Natriumhydroxid in 14 ml Wasser gelöst. Nach vollständiger Lösung des Produktes werden 3,9 g (0,028 mol) Methyljodid zugetropft. Man lässt bei Raumtemperatur über Nacht rühren. Danach wird der entstandene Feststoff abgesaugt, mit Wasser gewaschen und getrocknet. Nach säulenchromatographischer Reinigung erhält man 4 g (73% der Theorie) 6-Fluor-tert.-butyl-4-methyl-3-methylthio-1,2,4-triazin-5-on vom Schmelzpunkt 102-104°C.

In analoger Weise und entsprechend dem erfindungsgemässen Verfahren werden die in der nachfolgenden Tabelle 1 aufgeführten Verbindungen der allgemeinen Formel (I)

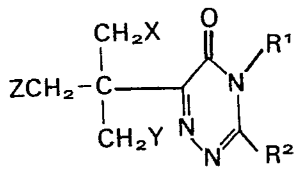

(I)

erhalten:

*Tabelle 1*

| Beispiel Nr. | X | Y | Z | $R^1$ | $R^2$ | Schmelzpunkt Fp (°C) |
|---|---|---|---|---|---|---|
| 5 | F | H | H | $NH_2$ | $-SC_2H_5$ | 80-83 |
| 6 | Cl | H | H | $NH_2$ | $-SC_2H_5$ | 93-96 |
| 7 | F | F | H | $NH_2$ | $-SCH_3$ | 122-124 |
| 8 | F | F | H | $NH_2$ | $-SC_2H_5$ | 100-103 |
| 9 | F | F | H | $NH_2$ | $-SC_3H_7$ | 110-112 |
| 10 | F | H | H | $NH_2$ | $-NHCH_3$ | 210-212 |
| 11 | F | H | H | $NH_2$ | $-NHC_2H_5$ | 118-120 |
| 12 | Cl | H | H | $NH_2$ | $-NHCH_3$ | 137-140 |
| 13 | Cl | H | H | $NH_2$ | $-N(CH_3)_2$ | 90-91 |
| 14 | Cl | H | H | $NH_2$ | $-NHC_2H_5$ | 103-106 |
| 15 | F | F | H | $NH_2$ | $-NHCH_3$ | 199-200 |
| 16 | F | F | H | $NH_2$ | $-N(CH_3)_2$ | 92-94 |
| 17 | F | F | H | $NH_2$ | $-NHC_2H_5$ | 146-148 |
| 18 | Cl | H | H | $NH_2$ | $-NHC_3H_7$ | 96-98 |
| 19 | Br | H | H | $NH_2$ | $-SCH_3$ | 109-110 |
| 20 | F | H | H | $NH_2$ | $-NHC_3H_7$ | 106-108 |
| 21 | F | F | H | $NH_2$ | $-NHC_3H_7$ | 70-72 |
| 22 | F | H | H | $NH_2$ | $-NH(CH_2)_5CH_3$ | 93-96 |
| 23 | Cl | H | H | $NH_2$ | $-NH(CH_2)_5CH_3$ | 99-104 |

*Verwendungsbeispiele*

In den nachfolgenden Beispielen wird die nachstehend angegebene Verbindung als Vergleichsubstanz eingesetzt:

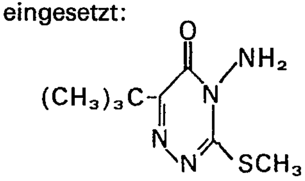

(A)

4-Amino-3-methylthio-6-tert.-butyl-1,2,4-triazin-5-on (bekannt z.B. aus US-PS 3671523).

*Beispiel A*

Pre-emergence-Test

Lösungsmittel: 5 Gewichtsteile Aceton
Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmässigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)

100% = totale Vernichtung

In diesem Test zeigen z.B. die Verbindungen gemäss Herstellungsbeispielen 1 und 2 bei vergleichbar guter Wirksamkeit eine bessere Selektivität in Mais, Baumwolle und Weizen als die aus dem Stand der Technik bekannte Verbindung (A).

*Beispiel B / Feldversuche*

Pre-emergence-Test / Freiland

Lösungsmittel: 5 Gewichtsteile Aceton
Emulgator: 1 Gewichtsteil Alkylarylpolyglykoläther

Zur Herstellung einer zweckmässigen Wirkstoffzubereitung wurde 1 Gewichtsteil Wirkstoff mit der angegebenen Menge Lösungsmittel vermischt, die angegebene Menge Emulgator zugegeben und das Konzentrat mit Wasser auf die gewünschte Konzentration verdünnt.

Kurz nach dem Aussäen der Testpflanzen im Freiland wurden die einzelnen Parzellen mit einer solchen Menge der Wirkstoffzubereitung begossen, wie für eine gleichmässige Benetzung der Bodenfläche erforderlich war. Die Wirkstoffkonzentration in der Zubereitung spielt dabei keine Rolle, entscheidend ist nur die Aufwandmenge des Wirkstoffes pro Flächeneinheit.

Nach 5 Wochen wurde der Schädigungsgrad der Testpflanzen bonitiert in % Schädigung im Vergleich zur Entwicklung der unbehandelten Kontrolle. Es bedeuten:

0% = keine Wirkung
100% = totale Vernichtung

In diesem Test zeigt insbesondere die erfindungsgemässe Verbindung (1) bei gleich guter Wirksamkeit eine wesentlich bessere Selektivität in Mais als die aus dem Stand der Technik bekannte Verbindung (A).

## Patentansprüche

1. Substituierte 6-Halogen-tert.-butyl-1,2,4-triazin-5-one der allgemeinen Formel (I)

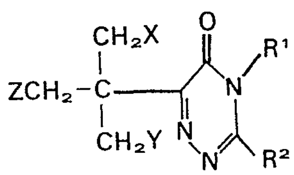 (I)

in welcher
$R^1$ für Amino oder Methyl steht,
$R^2$ für $C_{1-4}$-Alkylmercapto, $C_{1-4}$-Alkylamino oder Di-($C_{1-4}$)-alkylamino steht,
X für Halogen steht, und
Y und Z für Wasserstoff oder Halogen stehen.

2. Substituierte 6-Halogen-tert.-butyl-1,2,4-triazin-5-one der allgemeinen Formel (I) gemäss Anspruch 1, dadurch gekennzeichnet, dass
$R^1$ für Amino oder Methyl steht,
$R^2$ für Methylmercapto, Ethylmercapto, Propylmercapto, Methylamino, Ethylamino, Dimethylamino, Diethylamino oder Methylethylamino steht,
X für Fluor, Chlor oder Brom steht, und
Y und Z für Wasserstoff, Fluor, Chlor oder Brom stehen.

3. 4-Amino-6-chlor-tert.-butyl-3-methylmercapto-1,2,4-triazin-5-on der Formel

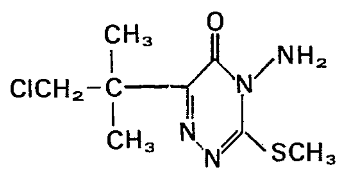 (1)

gemäss Anspruch 1.

4. 4-Amino-6-fluor-tert.-butyl-3-methylmercapto-1,2,4-triazin-5-on der Formel

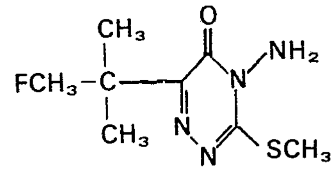 (2)

gemäss Anspruch 1.

5. Verfahren zur Herstellung von substituierten 6-Halogen-tert.-butyl-1,2,4-triazon-5-onen der Formel (I) gemäss Anspruch 1, dadurch gekennzeichnet, dass man in einer ersten Stufe Halogenpivaloylcyanide der Formel

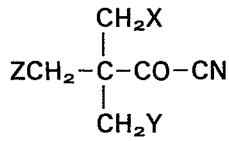 (II)

in welcher
X, Y und Z die oben angegebene Bedeutung haben,
gegebenenfalls in Gegenwart einer flüssigen Carbonsäure als Lösungsmittel, mit einer anorganischen Säure umsetzt und die hierbei entstehenden halogenierten Trimethylbrenztraubensäureamide der Formel

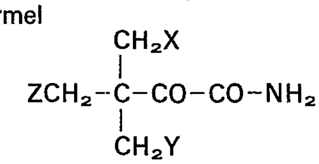 (IIIa)

in welcher
X, Y und Z die oben angegebene Bedeutung haben,
in einer zweiten Stufe in an sich bekannter Weise entweder direkt in der anfallenden Lösung oder nach Zwischenisolierung, gegebenenfalls nach vorheriger Verseifung zu den freien halogenierten Trimethylbrenztraubensäuren der Formel

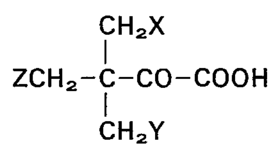 (IIIb)

in welcher
X, Y und Z die oben angegebene Bedeutung haben, mit Verbindungen der Formel

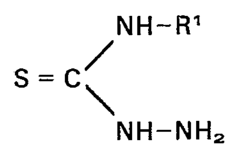 (IV)

in welcher
$R^1$ die oben angegebene Bedeutung hat,
in wässeriger bzw. in wässerig-saurer Lösung, gegebenenfalls in Gegenwart eines organischen Verdünnungsmittels, zu 6-Halogen-tert.-butyl-3-mercapto-1,2,4-triazin-5-onen der Formel

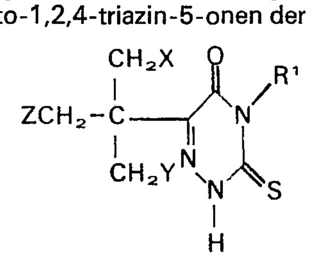 (V)

in welcher

$R^1$, X, Y und Z die oben angegebene Bedeutung haben, umsetzt, diese in einer dritten Stufe in bekannter Weise in alkalischer Lösung mittels Alkylhalogenid, vorzugsweise Alkyljodid oder -bromid, zu 6-Halogen-tert.-butyl-1,2,4-triazin-5-onen der Formel

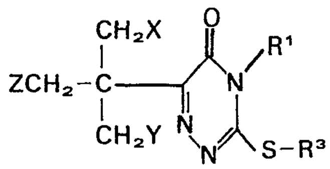 (Ia)

in welcher

$R^1$, X, Y und Z die oben angegebene Bedeutung haben, und

$R^3$ für Alkyl, vorzugsweise mit 1 bis 4 Kohlenstoffatomen, steht,

alkyliert und diese gegebenenfalls in einer vierten Stufe mit Aminen der Formel

$$HNR^4R^5 \qquad (VI)$$

in welcher

$R^4$ für Wasserstoff oder Alkyl, vorzugsweise mit 1 bis 4 Kohlenstoffatomen, steht, und

$R^5$ für Alkyl, vorzugsweise mit 1 bis 4 Kohlenstoffatomen, steht,

in Gegenwart eines Verdünnungsmittels umsetzt.

6. Herbizide Mittel, gekennzeichnet durch einen Gehalt an substituierten 6-Halogen-tert.-butyl-1,2,4-triazin-5-onen der Formel (I) gemäss Anspruch 1.

7. Verfahren zur Bekämpfung von unerwünschtem Pflanzenwachstum, dadurch gekennzeichnet, dass man substituierte 6-Halogen-tert.-butyl-1,2,4-triazin-5-one der Formel (I) gemäss Anspruch 1 auf die unerwünschten Pflanzen oder ihren Lebensraum einwirken lässt.

8. Verwendung von substituierten 6-Halogen-tert.-butyl-1,2,4-triazin-5-onen der Formel (I) gemäss Anspruch 1 zur Bekämpfung von unerwünschtem Pflanzenwachstum.

9. Verfahren zur Herstellung von herbiziden Mitteln, dadurch gekennzeichnet, dass man substituierte 6-Halogen-tert.-butyl-1,2,4-triazin-5-one der Formel (I) gemäss Anspruch 1 mit Streckmitteln und/oder oberflächenaktiven Mitteln vermischt.

## Claims

1. Substituted 6-halogeno-tert.-butyl-1,2,4-triazin-5-ones of the general formula (I)

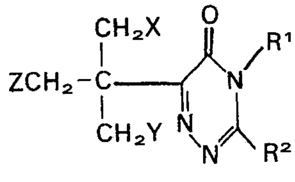 (I)

in which

$R^1$ represents amino or methyl,

$R^2$ represents $C_{1-4}$-alkylmercapto, $C_{1-4}$-alkylamino or di-($C_{1-4}$)-alkylamino,

X represents halogen, and

Y and Z represent hydrogen or halogen.

2. Substituted 6-halogeno-tert.-butyl-1,2,4-triazin-5-ones of the general formula (I) according to Claim 1, characterised in that

$R^1$ represents amino or methyl,

$R^2$ represents methylmercapto, ethylmercapto, propylmercapto, methylamino, ethylamino, dimethylamino, diethylamino or methylethylamino,

X represents fluorine, chlorine or bromine, and

Y and Z represent hydrogen, fluorine, chlorine or bromine.

3. 4-Amino-6-chloro-tert.-butyl-3-methylmercapto-1,2,4-triazin-5-one of the formula

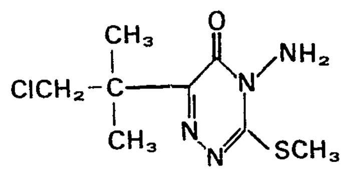 (1)

according to Claim 1.

4. 4-Amino-6-fluoro-tert.-butyl-3-methylmercapto-1,2,4-triazin-5-one of the formula

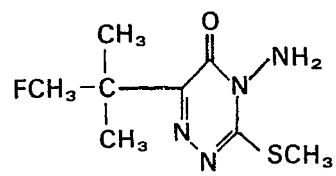 (2)

according to Claim 1.

5. Process for the preparation of substituted 6-halogeno-tert.-butyl-1,2,4-triazon-5-ones of the formula (I) according to Claim 1, characterised in that, in a first stage, halogenopivaloyl cyanides of the formula

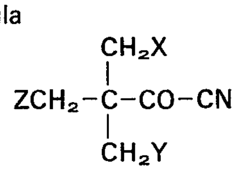 (II)

in which

X, Y and Z have the abovementioned meaning, are reacted with an inorganic acid, if appropriate in the presence of a liquid carboxylic acid, as the solvent, and the halogenated trimethylpyruvic acid amides thereby formed, of the formula

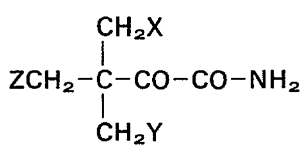 (IIIa)

in which

X, Y and Z have the abovementioned meaning, are reacted in a second stage, either directly in the solution obtained or after intermediate isolation, if appropriate after prior hydrolysis to give the free halogenated trimethylpyruvic acids of the formula

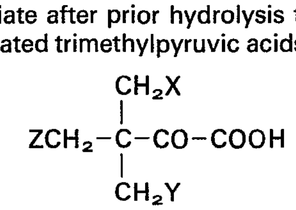 (IIIb)

in which

X, Y and Z have the abovementioned meaning, with compounds of the formula

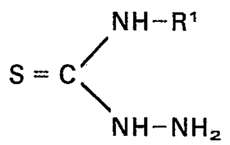

(IV)

in which

$R^1$ has the abovementioned meaning,

in a manner which is in itself known in aqueous or in aqueous-acid solution, if appropriate in the presence of an organic diluent, to give 6-halogeno-tert.-butyl-3-mercapto-1,2,4-triazin-5-ones of the formula

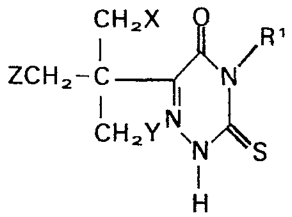

(V)

in which

$R^1$, X, Y and Z have the abovementioned meaning, these compounds are alkylated in a third stage by means of an alkyl halide, preferably an alkyl iodide or bromide, in a known manner in alkaline solution to give 6-halogeno-tert.-butyl-1,2,4-triazin-5-ones of the formula

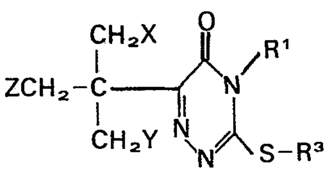

(Ia)

in which

$R^1$, X, Y and Z have the abovementioned meaning, and

$R^3$ represents alkyl, preferably with 1 to 4 carbon atoms,

and, if desired, these compounds are reacted in a fourth stage with amines of the formula

$$HNR^4R^5 \quad \text{(VI)}$$

in which

$R^4$ represents hydrogen or alkyl, preferably with 1 to 4 carbon atoms, and

$R^5$ represents alkyl, preferably with 1 to 4 carbon atoms,

in the presence of a diluent.

6. Herbicidal agents, characterised in that they contain substituted 6-halogeno-tert.-butyl-1,2,4-triazin-5-ones of the formula (I) according to Claim 1.

7. Process for combating undesired plant growth, characterised in that substituted 6-halogeno-tert.-butyl-1,2,4-triazin-5-ones of the formula (I) according to Claim 1 are allowed to act on the undesired plants or their environment.

8. Use of substituted 6-halogeno-tert.-butyl-1,2,4-triazin-5-ones of the formula (I) according to Claim 1 for combating undesired plant growth.

9. Process for the preparation of herbicidal agents, characterised in that substituted 6-halogeno-tert.-butyl-1,2,4-triazin-5-ones of the formula (I) according to Claim 1 are mixed with extenders and/or surface-active agents.

## Revendications

1. 6-Halogéno-tertio-butyl-1,2,4-triazine-5-ones substituées de formule générale (I)

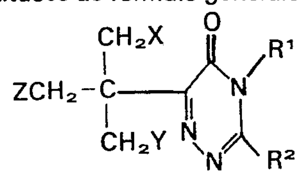

(I)

dans laquelle

$R^1$ est un groupe amino ou méthyle,

$R^2$ est un groupe alkylmercapto en $C_1$ à $C_4$, alkylamino en $C_1$ à $C_4$ ou di-(alkyle en $C_1$ à $C_4$)-amino,

X est un halogène, et

Y et Z représentent l'hydrogène ou un halogène.

2. 6-Halogéno-tertio-butyl-1,2,4-triazine-5-ones substituées de formule générale (I) suivant la revendication 1, caractérisées en ce que

$R^1$ est un groupe amino ou méthyle,

$R^2$ est un groupe méthylmercapto, éthylmercapto, propylmercapto, méthylamino, éthylamino, diméthylamino, diéthylamino ou méthyléthylamino,

X est le fluor, le chlore ou le brome, et

Y et Z représentent l'hydrogène, le fluor, le chlore ou le brome.

3. 4-Amino-6-chloro-tertio-butyl-3-méthylmercapto-1,2,4-triazine-5-one de formule

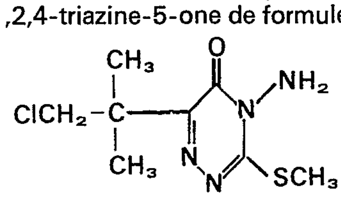

(1)

suivant la revendication 1.

4. 4-Amino-6-fluoro-tertio-butyl-3-méthylmercapto-1,2,4-triazine-5-one de formule

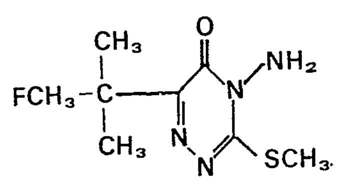

(2)

suivant la revendication 1.

5. Procédé de production de 6-halogéno-tertio-butyl-1,2,4-triazine-5-ones substituées de formule (I) suivant la revendication 1, caractérisé en ce qu'on fait réagir dans une première étape des cyanures d'halogénopivaloyle de formule

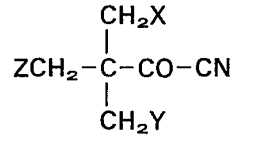

(II)

dans laquelle

X, Y et Z ont la définition indiquée ci-dessus, éventuellement en présence d'un acide carboxylique liquide utilisé comme solvant, avec un acide inorganique, et on fait réagir les amides d'acide triméthylpyruvique halogénés ainsi formés, de formule

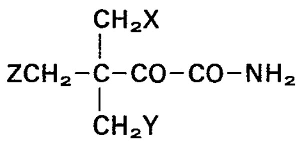

(IIIa)

dans laquelle

X, Y et Z ont la définition indiquée ci-dessus, dans une seconde étape d'une manière connue soit directement dans la solution produite, soit après isolement intermédiaire, éventuellement après saponification préalable en les acides triméthylpyruviques halogénés libres de formule

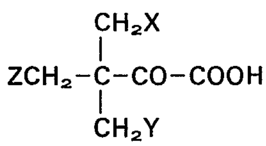

(IIIb)

dans laquelle

X, Y et Z ont la définition indiquée ci-dessus, avec des composés de formule

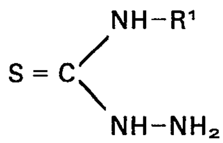

(IV)

dans laquelle

$R^1$ a la définition indiquée ci-dessus, en solution aqueuse ou en solution acide aqueuse, éventuellement en présence d'un diluant organique, pour former les 6-halogéno-tertio-butyl-3-mercapto-1,2,4-triazine-5-ones de formule

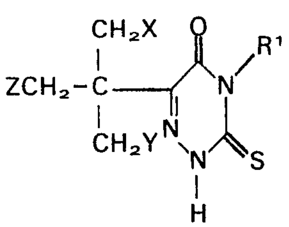

(V)

dans laquelle

$R^1$, X, Y et Z ont la définition indiquée ci-dessus, on alkyle ces dernières dans une troisième étape d'une manière connue en solution alcaline au moyen d'un halogénure d'alkyle, de préférence l'iodure ou le bromure d'alkyle, en les 6-halogéno-tertio-butyl-1,2,4-triazine-5-ones de formule

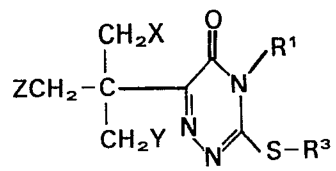

(Ia)

dans laquelle

$R^1$, X, Y et Z ont la définition indiquée ci-dessus, et

$R^3$ est un groupe alkyle, ayant de préférence 1 à 4 atomes de carbone,

et on fait éventuellement réagir ces dernières dans une quatrième étape avec des amines de formule

$$NHR^4R^5 \qquad (VI)$$

dans laquelle

$R^4$ est l'hydrogène ou un groupe alkyle, ayant de préférence 1 à 4 atomes de carbone, et

$R^5$ est un groupe alkyle, ayant de préférence 1 à 4 atomes de carbone,

en présence d'un diluant.

6. Compositions herbicides, caractérisées par une teneur en 6-halogéno-tertio-butyl-1,2,4-triazine-5-ones substituées de formule (I) suivant la revendication 1.

7. Procédé pour combattre la croissance d'herbes indésirables, caractérisé en ce qu'on fait agir des 6-halogéno-tertio-butyl-1,2,4-triazine-5-ones substituées de formule (I) suivant la revendication 1 sur les plantes indésirables ou sur leur milieu.

8. Utilisation de 6-halogéno-tertio-butyl-1,2,4-triazine-5-ones substituées de formule (I) suivant la revendication 1 pour combattre la croissance de plantes indésirables.

9. Procédé de préparation de compositions herbicides, caractérisé en ce qu'on mélange des 6-halogéno-tertio-butyl-1,2,4-triazine-5-ones substituées de formule (I) suivant la revendication 1 avec des diluants et/ou des agents tensio-actifs.